# EUROPEAN PATENT APPLICATION

(11) **EP 4 299 042 A1**
(43) Date of publication of application: **03.01.2024**
(21) Application number: 22182393.3
(22) Date of filing: 30.06.2022
(51) Int. Cl.: A61F 7/00, A61F 7/02, H04R 1/10, H04R 5/033

(54) **AN EARPHONE OR EARPIECE FOR CALORIC VESTIBULAR STIMULATION**

(71) Applicant: Scheuerer, Joachim, 7450 Tiefencastel (CH)
(72) Inventor: SCHEUERER, Joachim, 7450 Tiefencastel (CH); BRUGGER, Peter, 7317 Valens (CH)
(74) Representative: Grosfillier, Philippe

(57) **Abstract**

An ear stimulation device for caloric vestibular stimulation (CVS) to an ear of a wearer, including a headband, first and second earpieces connected to the headband, a blower device arranged at the headband, first and second fluidic ducts providing for a fluidic connection between the blower device and the two earpieces, and first and second thermoelectric cooling devices fluidically arranged between the blower device and the two earpieces configured to cool a fluid arriving from the blower device.

## Description

### FIELD OF THE INVENTION

The present invention is directed to the field of portable or wearable devices for caloric vestibular stimulation (CVS), and the field of audio headphones, earphones, earbuds, headsets, stetoclips, earplugs, or other wearable audio devices capable of providing cooling or heating to one or both ears of a wearer.

### BACKGROUND ART

In the field of headphones, earphones, earbuds, headsets, stetoclips, earplugs, or other wearable audio devices that can provide for audio signals to one or both ears of a wearer of the wearable audio device, different solutions exist to provide for thermal control to provide for a cooling, heating, or general temperature control to a component of the wearable device, mostly to provide for an increased comfort to the wearer of the device.

For example, International Patent WO 2016/148316A1 describes a headphone capable of measuring a body temperature of the wearer and controlling temperature including a case part, a heat transfer unit, a heat transfer part, a cooling unit, and a speaker unit, where the heat transfer unit is located at the center of the speaker unit, and includes an extension part extending into the user's ear, and the speaker unit located inside the passage unit such that sound generated from speaker unit is transferred into the user's ear through the passage unit.

In the field of caloric vestibular stimulation (CVS), U.S. Patent No. 9,283,111 provides for an in-ear CVS stimulation device that can administer caloric stimulation to the ear canal of a subject. The in-ear device includes first and second earpieces configured to be insertable into the ear canals of the subject, at least first and second thermoelectric devices thermally coupled to respective ones of the first and second earpieces, a first heat sink thermally coupled to the first thermoelectric device opposite the first earpiece and a second heat sink thermally coupled to the second thermoelectric device opposite the second earpiece, and a controller with a waveform generator in communication with the first and second thermoelectric devices, the waveform generator configured to provide for the caloric vestibular stimulation of the subject.

However, despite all the advancements in the field of temperature controlled wearable audio devices, and in the field of wearable devices for caloric vestibular stimulation, advanced solutions are desired to combine the audio and the caloric vestibular stimulation capabilities, and to provide for a simplified and efficient devices and systems to incorporate these two features.

### SUMMARY

According to an aspect of the present invention, an in-ear stimulation system for caloric vestibular stimulation (CVS) to an ear of a wearer is provided, the system preferably including at least one earpiece, for example two earpieces, one for the left ear and one for the right ear of a wearer, and a device for holding the at least one earpiece. Moreover, preferably, the at least one earpiece includes a thermally conductive element having a contact surface and a protrusion configured to fit at least partially into an ear channel of the ear of the wearer. In addition, the device for holding the at least one earpiece includes a thermoelectric cooler or heater having a cooling or heating surface, and an energy supply device for providing electric energy to the thermoelectric cooler. Moreover, preferably, the in-ear stimulation system further includes a securing element for holding the at least one earpiece at the device for holding such that the contact surface of the thermally conductive element of the at least on earpiece is in contact with the cooling surface of the thermoelectric cooler, when the at least one earpiece is placed to the device for holding.

According to another aspect of the present invention, an ear stimulation device for caloric vestibular stimulation to an ear of a wearer is provided. Preferably, the ear stimulation device includes a headband, first and second earpieces held by the headband, a blower device arranged at the headband, first and second fluidic ducts providing for a fluidic connection between the blower device and the two earpieces, and first and second thermoelectric cooling or heating devices fluidically arranged between the blower device and the two earpieces, configured to cool a fluid arriving from the blower device.

According to still another aspect of the present invention, an in-ear stimulation system is provided, the in-ear stimulation system preferably including a data processing device having a first data communication interface, and an ear stimulation device for caloric vestibular stimulation (CVS) to an ear of a wearer. Moreover, preferably, the ear stimulation device includes first and second earpieces, a blower device, first and second fluidic ducts providing for a fluidic connection between the blower device and the two earpieces, first and second thermoelectric cooling or heating devices fluidically arranged between the blower device and the two earpieces configured to cool a fluid arriving from the blower device, and a second data communication interface for communicating with the first data communication interface of the data processing device.

According to yet another aspect of the present invention, an ear stimulation system is provided, including a data processing device having a first data communication interface and a display screen, and at least one ear stimulation device for caloric vestibular stimulation (CVS) to an ear of a wearer, the ear stimulation device preferably including at least one earpiece means for cooling or heating an ear channel of a wearer, and a second data communication interface for communicating with the first data communication interface of the data processing device, wherein the data processing device is configured provide for at least one of a temperature set value or a temperature profile to the at least one ear stimulation device for cooling or heating an ear channel of a wearer.

The above and other objects, features and advantages of the present invention and the manner of realizing them will become more apparent, and the invention itself will best be understood from a study of the following description with reference to the attached drawings showing some preferred embodiments of the invention.

### BRIEF DESCRIPTON OF THE SEVERAL VIEWS OF THE DRAWINGS

The accompanying drawings, which are incorporated herein and constitute part of this specification, illustrate the presently preferred embodiments of the invention, and together with the general description given above and the detailed description given below, serve to explain features of the invention.

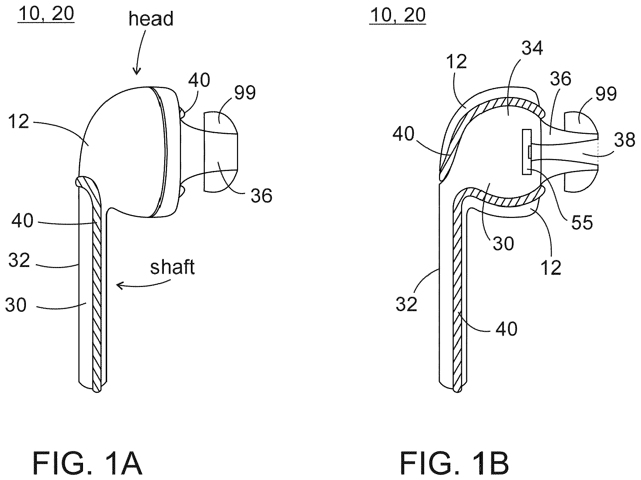

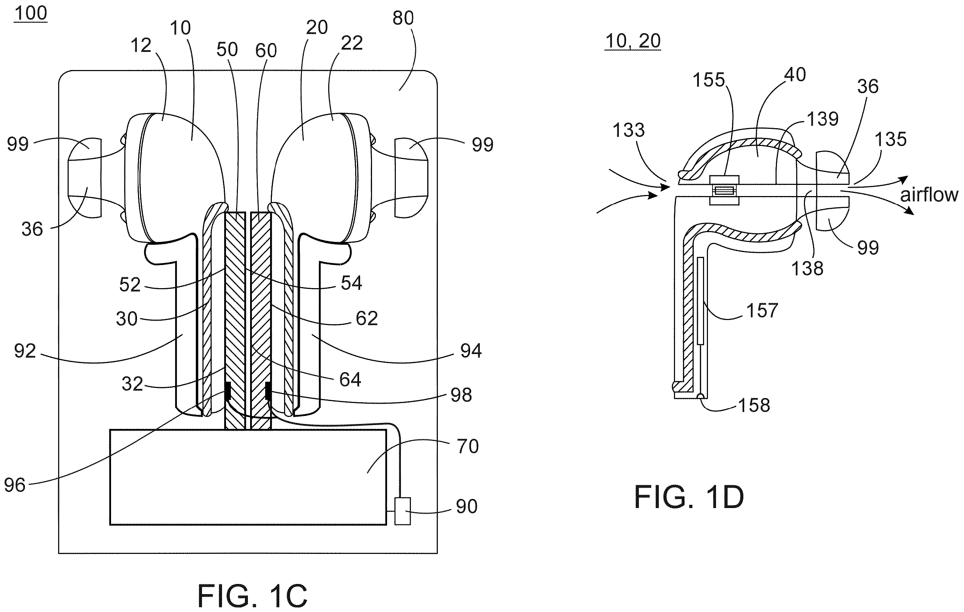

FIGs. 1A to ID show different views of a schematic and exemplary representation of an in-ear stimulation system 100 for caloric vestibular stimulation (CVS) to an ear of a wearer according to an aspect of the present invention, the system 100 including at least one earpiece 10, 20, for example two earpieces for the left and right ear of a wearer, and a device 80 for holding the at least one earpiece 10, 20 for thermally charging or cooling a thermally conductive element 30, with FIG. 1A showing an exemplary side view of an earpiece 10, 20, FIG. 1B showing a cross-sectional view of an earpiece 10, 20 with a thermally conductive element 30 arranged therein, FIG. 1C showing two earpieces 10, 20 that are placed on a holder 80 for actively cooling the thermally conductive element 30 with an thermoelectric cooler system 50, 60, and FIG. ID showing a variant with a cross-sectional view of an earpiece 10, 20;

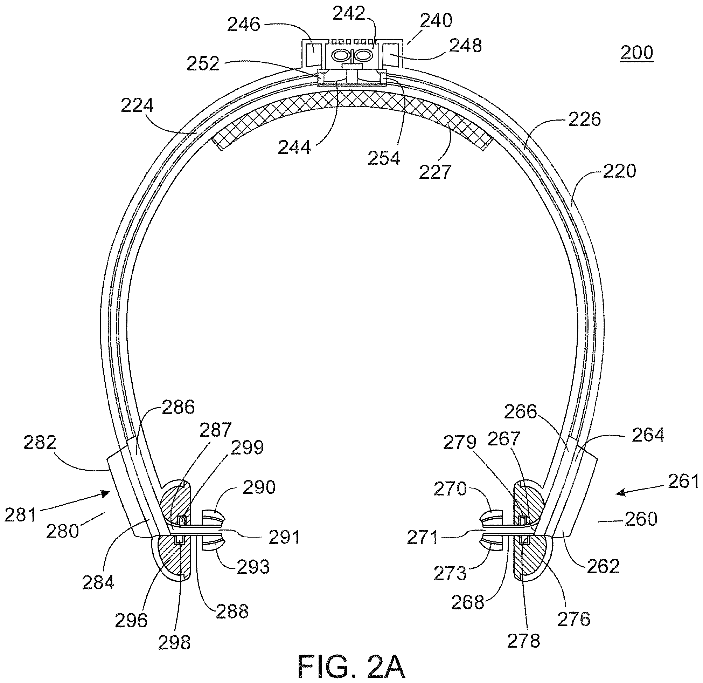

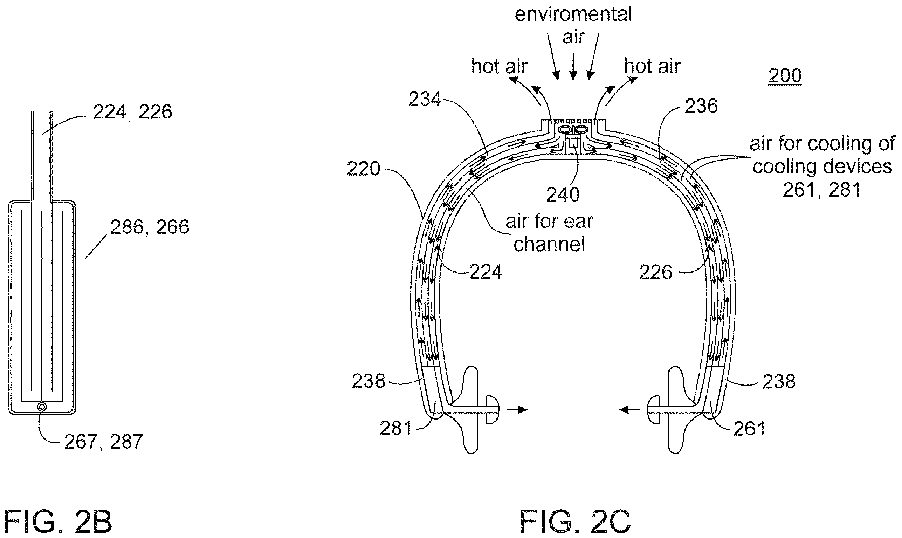

FIGs. 2A to 2C show different views of a schematic and exemplary representation of an in-ear stimulation device or system 200 for CVS according to another aspect of the present invention, showing a headset-type CVS device with two earpieces 260, 280, a fluid or air blowing unit 240, a headband 220, with FIG. 2A showing an exemplary cross-sectional view thereof, and FIG. 2B showing a detailed cross-sectional view of the cooling chamber 266, 286 of the first and second thermoelectric cooling devices 261, 281 that can be interconnected with a thermoelectric cooler 264, 284, and FIG. 2C showing an exemplary cross-sectional view of an in-ear stimulation device or system 200 with encapsulated heat-sink or heated surfaces with fluidic return lines 234, 236 for evacuating generated heat from an area of an ear of the wearer;

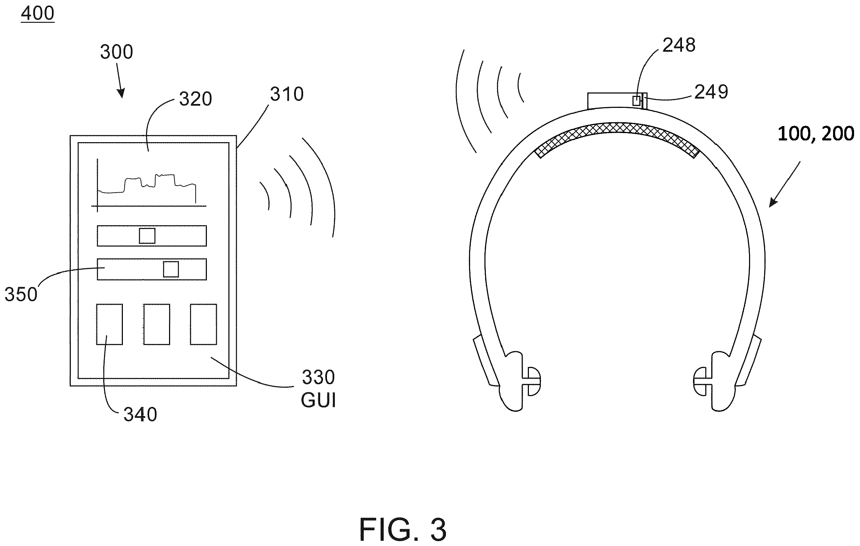

FIG. 3 shows an overview of a system 400 for performing CVS, with a smartphone, tablet, or other computing device 300 having a display screen 320 interconnected to in-ear stimulation system 200 for control and for providing data to computing device 300 via a data communication interface; and

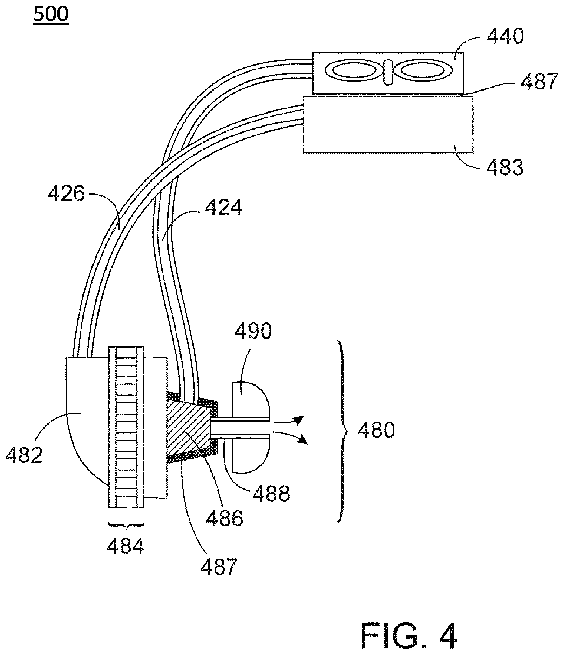

FIG. 4 shows a partial and cross-sectional view of another embodiment of a portion of an in-ear stimulation device or system 500 as a headset-type CVS device, using a central heatsink 483 that is interconnected to thermoelectric cooling element 484 via a heat transfer device 426, for example a heat pipe.

Herein, identical reference numerals are used, where possible, to designate identical elements that are common to the figures. Also, the images in the drawings are simplified for illustration purposes and may not be depicted to scale.

### DESCRIPTION OF THE SEVERAL EMBODIMENTS

According to an aspect of the present invention, an in-ear stimulation system 100 for caloric vestibular stimulation (CVS) to an ear of a wearer is provided, as shown in FIGs. 1A to ID. For example, as shown in FIG. 1C, system 100 can include at least one earpiece 10, 20, for example two separate earpieces 10, 20 for the left and right ear of a wearer that can cool down an ear channel of the wearer of the earpiece 10, 20, and a device 80 for holding the at least one earpiece 10, 20 for thermally charging, for example for cooling or heating a thermally conductive element 30 of the earpiece 10, 20, according to one aspect of the present invention. In the variant shown, earpieces 10, 20 can be separate elements, but it is also possible that the earpieces 10, 20 are removably or fixedly interconnected to a headband to form a headphone-like device, or a headset. With this in-ear stimulation system 100, thermally conductive element 30 of the at least one earpiece 10, 20 can be cooled down or heated up to a desired temperature when they are placed or otherwise interconnected to the holder 80, by the use of a thermoelectric cooler system 50, 60 of holder 80. The thermally conductive element 30 can have a certain thermal storage capacity or specific heat capacity for holding thermal energy. Thereby, once removed from holder 80 and inserted into an ear of a wearer, the at least one earpiece 10, 20 can operate as a passive cooling or heating device for providing cooling to an ear channel of a wearer, for example with a protrusion, tube, or duct 36 of thermally conductive element 30 that can be at least partially inserted to an external auditory canal or meatus of an ear of a wearer. At least one earpiece is thereby not itself equipped with an active heating or cooling device, for example a thermoelectric cooler, but has been charged or loaded with thermal energy to provide for the cooling or heating for a certain time duration, before thermal recharging is needed.

Thermally conductive element 30 can be made of one continuous part of material that has a high thermal conductivity, and a high thermal storage capacity. For example, copper (Cu) or a copper alloy can be used, or other materials preferably having a thermal conductivity λ over 200 W/m · K and a specific heat capacity cₚ over 200 J/kg · K. Protrusion, tube, or duct 36 can also be removably or fixedly attached to and at least partially surrounded by an earbud or eartip 99, for example a soft, elastic plug-like device, for example having fluid pressure release channels. It is also possible that protrusion, tube, or duct 36 is a removable part that can be removed from the thermally conductive element 30, for example by a press-fit or clipping interconnection, for example for cleaning, sterilizing, or for replacing duct 36.

The device 80 for holding the at least one earpiece 10, 20 can have different shapes and arrangements, for example but not limited to a casing, case, enclosure, box, receptacle, open tray, closable tray, substrate and in the variant shown, and can have two Peltier elements 50, 60 as the thermoelectric cooler system, the Peltier elements 50, 60 being powered by an energy supply device 70, for example a rechargeable or non-rechargeable battery that can be removably or fixedly installed to the device 80 for holding the at least one earpiece 10, 20. In a variant, it is also possible that energy supply device 70 includes a power controller for providing electrical energy to the thermoelectric cooler system, and is connected to an external power supply, for example one that can be electrically connected to the power grid. Each Peltier element 50, 60 has a cooling surface 52, 62, that is arranged and shaped to come into contact with a contact surface 32 of the thermally conductive element 30 of the at least on earpiece 10, 20, while opposing surfaces 54, 64 of Peltier elements 50, 60 are heated. For example, the shape of contact surface 32 and cooling surfaces 52, 62 can be such that they are complementary shaped for easy interconnection. Device for holding 80 can also be equipped with one or more securing elements 92, 94 for securing the at least one earpiece 10, 20 at the device 80, such that the contact surface 32 of the thermally conductive element 30 of the at least on earpiece 10, 20 comes into and remains in contact with the cooling surface 52, 62 of the thermoelectric cooler system 50, 60, when the at least one earpiece 10, 20 is placed to the device 80.

Thermoelectric cooler system 50, 60 can be arranged in different ways, with the purpose to have cooling surface 52, 62 arranged to be exposed and configured to contact the contact surface 32 of thermally conductive element 30. In the variant shown, each thermoelectric cooler system 50, 60 is a Peltier element of longitudinal bar-like shape, with the heating surfaces 54, 64 facing each other, and the cooling surfaces 52, 62 facing away from each other. For example, for heat evacuation from device 80, it is possible to have an air flow channel or a heat-sink sandwiched between the two heating surfaces 54, 64 of the bar-like Peltier elements 50, 60. It is also possible the thermoelectric cooler system 50, 60 is formed as a plate as a single Peltier element, with the cooling surfaces 52, 62 being two or a single exposed areas of the cooling side of the Peltier element, and the heated side of the Peltier element with two or a single heated surfaces 54, 64 forming part of an external wall of the device 80 for holding, for example with or without an additional heat sink.

In a variant, it is possible that an electric device is provided between energy supply device 70 and the one or more Peltier elements 50, 60 that can be controlled to reverse a polarity of the voltage that is applied to one or more Peltier elements 50, 60, so that arrangement of the cooling versus heating surfaces 52, 62 and 54, 64 is reversed. For example, this can be done with a solid-state polarity inverter device, or other type of switching device that can be controlled by controller 90. Thereby, cooling surfaces 52, 62 that can be put into contact with one or more contact surfaces 32 can become a heating surface, so that thermally conductive element 30 can store thermal energy that can depart heat to an ear channel of the wearer, as compared to the environmental temperature.

As shown in FIG. 1A, in an exemplary embodiment, contact surface 32 of thermally conductive element 30 is such that is exposed from an outer surface of earpiece 10, 20, in the variant shown forming a longitudinal surface extending along an axis that is formed by a holding shaft of the respective earpiece 10, 20, for example forming a segment of a cylindrical surface, or a flat surface. In this variant, contact surface 32 is exposed to the air when worn by the wearer, and will not contact a skin at or around an ear of wearer, thereby avoiding a discomfort with unwanted cooling or heating of the skin, or an increased heating of thermal conductive element 30 by the warmth of the skin. Other arrangements of the contact surface 32 are also possible, for example but not limited to a semi-spherical surface at the head of the earpiece 10, 20, a circular disk or a flat surface having another shape at the head of the earpiece 10, 20, a surface covering both at least a portion of the head and the shaft. It is also possible that a cover is removably placeable on to contact surface 32 to provide for thermal insulation when the earpiece 10, 20 is in use.

In the variant shown in FIG. 1C, the securing elements 92, 94 can be walls that are shaped complementarily to a shaft portion of the earpiece 10, 20, permitting a placement of the shaft portion of earpiece 10, 20 between securing elements 92, 94 and the Peltier elements 50, 60, dimensioned and arranged such that the contact surface 32 of the thermally conductive element 30 comes into contact with cooling surfaces 52, 62 of Peltier elements 50, 60, respectively, thereby permitting a manual and removable interconnection to device 80, and allowing for thermal "charging" of the element 30 with thermoelectric system 50, 60. Securing elements 92, 94 can be embodied with various variants, for example as cavities or bores arranged in an exposed surface of the device for holding 80, having a complementary shape to the portion of the earpiece 10, 20 having the contact surface 32, clips or locking elements for removable attachment, as a lid of device for holding 80 to press earpieces 10, 20 and surface 32 towards the cooling surfaces 52, 62. It is also possible that securing elements 92, 94 are movable by a motorized device to press contact surface 32 and cooling surfaces 52, 62 together when earpieces 10, 20 are placed into or otherwise provided to the device for holding 80.

Moreover, the device for holding 80 can further include one or more temperature sensors 96, 98 for measuring, either directly or indirectly, the temperature of the thermally conductive element 30, the cooling or heating temperature provided by the thermoelectric cooler system 50, 60, for example by measuring a temperature of a surface of thermoelectric system 50, thermoelectric system 60 or both 50, 60, as illustrated exemplarily in FIG. 1C. For example, temperature sensors 96, 98 can be operatively interconnected to a controller 99, for example but not limited to a semi-conductor based microcontroller device 99, and can be arranged to measure a surface temperature of the cooling surfaces 52, 62 of the Peltier elements 50, 60, for example by arranging a small surface temperature sensor onto surfaces 52, 62 of Peltier elements 50, 60. This arrangement allows to perform an open-loop or closed-loop temperature controlled cooling or heating of the thermally conductive element 30, controlling the electric power provided to Peltier elements 50, 60 by energy storage device 70, for example in a modulated or on/off fashion with a power controller, to reach a desired temperature for thermally conductive element 30 of earpiece 10, 20. In addition, given the presence of one temperature sensor 96, 98 for each earpiece 10, 20 respectively, it is possible to selectively "charge" each earpiece 10, 20 with a different temperature, for different types of CVS applications.

As shown in FIGs. 1A and 1B, the earpiece 10, 20 can be made of an outer shell 12 that forms at least a part of the enclosure of the earpiece 10, 20, and a thermal insulation layer 40 can be arranged to provide for a thermal barrier between thermally conductive element 30 and casing 12, to avoid an increased re-heating or re-cooling of thermally conductive element 30 by the environmental temperature, and to avoid cooling or heating the casing 12 causing discomfort for the wearer, for example by cooling part of the head of earpiece 10, 20 that can be placed into the ear cavity or concha of the ear of the wearer. Thermal insulation layer 40 can be made of a layer having a low thermal conductivity, or a multiplayer structure including a metal layer and a synthetic insulation layer, for example but not limited to an epoxy layer, polyvinyl chloride (PVC), polyurethane, rubber, polystyrene material. For example, thermal insulation layer 40 can be coated around thermally conductive element 30 such that no part of casing 12, 22 comes into contact with thermally conductive element 30, forming a complete thermal barrier.

In a variant, the in-ear stimulation system 100 for caloric vestibular stimulation (CVS) can also be provided with one or more speaker or audio device 55, to combine audio or sound generation with the CVS experience for the user. For example, in a variant shown in FIG. 1B, protrusion, tube, or duct 36 of thermally conductive element 30 that protrudes from head of earpiece 10, 20 can be at least partially traversed by a bore or hole 38, and at a speaker device 55, for example a small sound chip, can be arranged to emit sound or audio into bore or hole 38, having an opening towards the ear canal of wearer. In this respect, protrusion, tube, or duct 36, or at least a part thereof, can act as a sound tube for propagating sound or acoustic waves, in addition to the cooling or heating. Speaker device 55 can include a wireless communication device to receive audio signals from an external apparatus, for example but not limited to a Bluetooth interface, an IrDA interface, WiFi interface, and can be powered by a rechargeable or removable energy supply device or battery 157, exemplarily shown in FIG. ID with an electric terminal 158 for charging the battery 157. It is also possible that no energy supply device 157 is provided, and the speaker device 55 can be connected with a wired connection to an external apparatus to receive audio or sound signals. In the variant shown, speaker device 55 is embedded into a cavity of thermally conductive element 30, with an audio diaphragm thereof arranged to be perpendicular to an axis of extension of bore or hole 38.

In another variant shown in FIG. ID, while still no active cooling device is present in the one or more earpiece 10, 20, a blower or fan device 155 is arranged to provide for an additional fluid flow to an ear channel of the wearer, via inlet opening 133 of duct 36, traversing channel, bore, or hole 138, and outlet opening 135. In the variant shown, fan device 155 is arranged inside traversing channel 138, but can also be arranged at the inlet opening 135. Also, traversing channel 138 can be configured for providing an increased cooling or heating surface to the moving fluid, for example by baffles, fins, meandering structure. In the context of the present invention, a fluid is preferable the air from the external environment of the earpieces 10, 20, but can also be a different kind of fluid, or a combination of environmental air and another fluid, for example another type of gaseous fluid, for example oxygen, hydrogen, carbon dioxide, ozone, argon, or a combination thereof, for example provided by a portable tank or reservoir to provide for the gas at air inlet opening 133 or channel 138. Fan device 155 can be an impeller in the traversing channel 138 arranged to traverse the head of earpiece 10, 20 from protrusion 36 to a rear or distal surface of the head, forming an air inlet opening 133 at the head and an air outlet opening 135 at the end of the protrusion 36.

Moreover, a release mechanism can be provided at holding device 80, configured to provide for an air gap or other distancing between the one or more contact surfaces 32 of earpieces 10, 20 and the one or more cooling surfaces 52, 62 of thermoelectric cooler system 50, 60, once a temperature of the contact surfaces 32 or the cooling surfaces 52, 62 has reached a predefined or stored temperature value. For example, such release mechanism can be implemented as motors, linear actuators, or snap-release mechanisms that are controllable by controller 90, configured to move securing elements 92, 94 away from the respective thermoelectric cooler system 50, 60, thereby releasing the earpieces 10, 20 from contact with the respective cooling surfaces 52, 62. In this respect, controller 90 can be configured or programmed to control the thermal cooling or heating of conductive element 30 of earpieces 10, 20 with coolers 50, 60, and with the help of the measurements by temperature sensors 96, 98, control the stopping of the cooling and the removal of earpieces 10, 20 from being in contact with coolers 50, 60 upon reaching a predefined temperature. In a variant, release mechanism can be implemented by an insertion ejection mechanism where earpieces 10, 20 can be inserted into a reversibly tiltable or retractable tray or loader that can be pressed to put earpieces 10, 20 in contact with the respective cooling surfaces 52, 62 by compressing a loading spring, and upon reaching the desired temperature, the tray or loader by be ejected or opened to release contact of earpieces 10, 20 with cooling surfaces 52, 62, for example by the energy of the compressed spring. Such release mechanism would permit to avoid a discharging or reheating of the thermally conductive elements 30, if after the cooling they would remain in contact with the cooling surfaces 52, 62 of the unpowered coolers 50, 60. Other release mechanism could include simple motors or actuators that can push earpieces 10, 20 away cooling surfaces 52, 62, for example by cam wheel or linear motion, to provide for the air gap.

In an exemplary embodiment, square-sized Peltier elements of the size 12 mm to 12 mm or 14 mm to 14 mm have been used, where the cooled side reached 6°C to 8°C and the heated side reached 38°C to 40°C after being powered for ten (10) seconds at an environmental temperature of 22°C, using a 3.3V Lithium-Ion battery as the energy storage device 70, and the battery capacity was sized to be able to keep these temperature values for approximately thirty (30) minutes. A temperature sensor 96, 98 in cooperation with a controller 90 can be configured to measure the temperature of thermally conductive element 30 of the respective earpiece 10, 20, and a signal can be generated upon reaching a desired temperature of the thermally conductive element 30, either for heating or for cooling, for example by an alerting means such as an audio speaker, a vibration device, light-emitting diode, display, or other means to alert the potential wearer or user of the readiness of the earpieces 10, 20.

According to another aspect of the present invention, an ear stimulation device or system 200 for CVS is provided, being a headset-type CVS device with two earpieces 260, 280, a blower device 240 for fluid, for example environmental air that is aspirated from the external environment, and a headband 220, with FIG. 2A showing an exemplary cross-sectional view thereof, and FIG. 2B showing a detailed cross-sectional view of the cooling chamber 266, 286 of the first and second thermoelectric cooling devices 261, 281 that can be interconnected with a thermoelectric cooler 264, 284.

The ear stimulation device or system 200 includes a headband 220 that can have fixed dimensions or can be extended and retracted to fit different head sizes of a wearer, first and second earpieces 260, 280 connected to the headband 220, for example at respective ends of the headband 220 with a fixed, articulable or removable interconnection, a blower device 240 arranged at the headband 220, for example at a top area of the headband 220 configured to pull in air from the external environment, preferably from an area above a head of the wearer and the headband 220, with at least one rotative fan 242, a bladeless fan or air multiplier, or a combination thereof. Preferably, blower device 240 is arranged at a location on the headband 220 that is to be substantially in a middle or halfway between the first and second earpieces 260, 280, configured to draw in the fluid, for example environmental air, from the external environment. In this respect, the middle of headband can be defined as arranged at equal distance or lengths between the first and second earpieces 260, 280, plus and minus 10% to 25% thereof, to still be located at a top of the head of a wearer. This allows to remove the blower device 240 away from the ears of the wearer, to reduce noise and vibrations at the ear of the wearer, and also allows to provide for a better weight distribution, where a weight of the blower device 240 is carried by the head of the wearer, and not by the ears. Also, as discussed above, the fluid is preferable the air from the external environment of ear stimulation device or system 200, but can also be a different kind of fluid, or a combination of environmental ear and another fluid, for example another type of gaseous fluid, for example oxygen, hydrogen, carbon dioxide, ozone, argon, or a combination thereof, for example provided by a portable tank or reservoir to an air inlet opening of blower device 240. Moreover, an energy supply device 246, for example one or more rechargeable batteries, non-rechargeable batteries, supercapacitor, including power control device, or other type of energy providing device can be arranged at the blower device 240, for example for powering the fan 242 of blower device 240 with electrical energy to further concentrate the weight to a top of ear stimulation device or system 200.

Moreover, device 200 can further include a first and a second fluidic ducts 224, 226 providing for a fluidic connection between the blower device 240 and the two earpieces 260, 280. For example, fluidic ducts 224, 226 can be made of a flexible tubing material that is arranged to either be inside headband 220, or arranged along headband, to lead to the two earpieces 260, 280, for example a thermoplastic tubular structure. At the blower device 240, a fluid distribution element 244 can be arranged to redirect a fluid flow from the fan 242 towards the inlets of both fluidic ducts 224, 226, and fluid distribution element 244 can also be equipped with two manually or automatically controllable valves 252, 254 to control a fluid flow from device 240 to the two fluidic ducts 224, 226 selectively, for example controllable with a data processing device 248. Data processing device 248 can be a conventional microcontroller that is arranged at device 200, for example in close proximity or integrated with blower device 240. In a variant, instead of two valves 252, 254, blower device 240 can include two different fans 242.1 and 242.2 to selectively control and deliver air to the fluid ducts 224, 226 selectively.

Also, device 200 can further include first and second thermoelectric cooling devices 261, 281 fluidically arranged between the blower device 240 and the two earpieces 260, 280 configured to cool or heat the arriving fluids, for example two air streams, arriving from the blower device 240 via fluidic ducts 224, 226, and thereafter delivered to each ear of wearer for CVS stimulation. In the variant shown, first and second thermoelectric cooling devices 261, 281 are shown to be somewhat integrated to the respective first and second earpieces 260, 280, but can also be arranged further up along the headband 220, but preferably closer to earpieces 260, 280 than to blower device 240 along the respective fluidic pathway. First and second thermoelectric cooling devices 261, 281 can be electrically interconnected with a cable or wiring to energy supply device 246 via the headband 220, and their operation can be controlled by data processing device 240.

Preferably, each one of the first and second thermoelectric cooling devices 261, 281 includes a thermoelectric cooling element 264, 284, for example a planarly-shaped or curve or arcuate-shaped Peltier element having a heated side facing away from the head of wearer and having a cooled side facing towards the head of wearer, a heat sink 262, 282 having a first inner surface in contact with a heated side of the thermoelectric cooler 264, 284, and a second surface exposed to an external environment. The second surface that is exposed to the external environment can be equipped with a plurality of fins, baffles, or other surface-increasing structures for efficient cooling of the heated side. At the cooled side of the thermoelectric cooler 264, 284, first and second cooling chambers 266, 286 can be arranged having a fluid inlet for receiving an incoming fluid from the first and second fluidic ducts 224, 226, and a fluid outlet for releasing a cooled outgoing fluid towards the earpiece 260, 280. Cooling chambers 266, 286 are configured to, after receiving fluid blown by blower device 240 via ducts 224, 226, to at least one of (i) increase a fluid passage time of a fluid passing through chambers 266, 286 as compared to a more direct fluidic connection, or (ii) increase a cooling surface of the fluid passing through chambers 266, 286 as compared to a cooled surface of thermoelectric cooler 264, 284, or both (i) and (ii).

Moreover, different types of sensors 279, 299 can be arranged for each earpiece 260, 280, for example temperature sensors that are configured to measure a temperature of the fluid that is provided to fluid channel 268, 288 and delivered to the ears of the wearer, and to provide for data on the measured temperature to data processing device 248. Thereby, it is possible that a closed-loop temperature control of thermoelectric cooler 264, 284 is performed with data processing device, for example by using a proportional P controller, proportional-integral PI controller, or a proportional-integral-derivative PID controller, for electrically controlling the thermoelectric coolers 264, 284 by data processing device 248. For example, the power supply to thermoelectric coolers 264, 284 can be modulated by a controllable modulation scheme, for example a pulse width modulation (PWM). It is also possible that the temperature control is performed via an external device, for example a data processing device 300 in communicative connection with device 200 as further explained below with respect to FIG. 3. Temperature sensors can also be used to limit a temperature of the fluid to a maximal or minimally permissible temperature for safety and comfort, by using data processing device 248 to control or limit the temperature to a predefined or stored value.

Moreover, sensors 279, 299 can also include one or more presence or absence sensors that can detect whether fluid channels 268, 288 are arranged to protrude into the ears of a wearer, for example by electrodes that can perform capacitive measurements, optical sensors to detect presence of the skin, or other types of presence or distance measurement sensors. This can be used by data processing device 248 to turn the cooling/heating on or off, for example to turn the cooling off when device 200 is removed from the head of the user. Other sensors 279, 299 that can be arranged in fluid channel 268, 288 or elsewhere in earpieces 260, 280 can be pressure sensors configured to measure a pressure of the inside the fluidic system, for example a fluidic pressure inside fluid channels 268, 288, for example with the goal to avoid the build-up of an overpressure inside the ear channel of the wearer, for example to control a speed of rotation of the fan or ventilator 242 of blower device 240 to thereby control a rate of fluidic flow. This can also be done in a closed-loop controlled fashion. Different wearers of the device 200 may have different sensibility levels to air pressure in their ear channel, and with this aspect of pressure control, different set values can be provided. As the fluid flow velocity that exits first and second outlets 271, 291 can have an impact on both the temperature and the pressure inside the ear channel of wearer, it is possible that both the pressure and the temperature are controlled together in a closed loop fashion, for example by the use a prestored relationship table that defines the relationship of different fluid flow speeds and different cooling temperatures on the in-ear temperature and in-ear pressure, to provide for different set values for temperature and fluidic velocity control.

In a variant, it is possible that first and second earplug or eartip 270, 290 have one or more sensors 279, 299 including an ear-electroencephalography (EEG) sensor that is in operative interconnection with data processing device 248, the ear-EEG sensor having a plurality of electrodes for sensing signals in the ear canal, for example different sensor electrodes that are arranged at a circumference of the first and second earplugs or eartip 270, 290, for example configured to detect magnetoencephalograph (MEG) signals. For example, the ear-EEG sensors could be similar to ones described in U.S. Patent Publication No. 2021/0000370, this patent publication herewith incorporated by reference in its entirety, but requiring a central fluid channel 268, 288 arranged therethrough. Data from the ear-EEG sensors can be provided to an external data processing device, such as the exemplary variant described in FIG. 3 below, for additional processing.

In a variant, as shown in FIG, 2C, instead of having heat sinks 262, 282 with exposed surface for evacuating the heat generated by the thermoelectric coolers 264, 284 at first and second earpieces 260, 280, or in conjunction with heat sinks 262, 282, it is possible that there is a separate fluidic line is arranged from blower device 242, leading to heat sinks 262, 282, or the heated surface of thermoelectric coolers 264, 284, and back to blower device 240 or other location away from earpieces 260, 280, one each in parallel to first and second fluidic ducts 224, 226. Such fluidic return lines 234, 236 from blower device 240 to the thermoelectric coolers 264, 284 and back can allow for an encapsulation of the heat sinks 262, 282 or the thermoelectric coolers 264, 284, for example with a heat insulating material and an outer casing shell or thermal encapsulation layer 238, to thereby avoid any heated zone close to the ears of the wearer. It possible that blower device 240 has two different ventilators or fans, one for the first and second fluidic ducts 224, 226, and one for the first and second fluidic return lines 234, 236. This embodiment would allow to provide for a cooling fluid, for example air, to be provided from blower device 240, without having any hot or heated exposed surfaces that need cooling at a proximity of the ears of the wearer, for example by heat sinks 262, 282 that could be easily touched by the wearer.

FIG. 2B shows an exemplary embodiment of where chambers 266, 286 are provided with two meandering or spiraling air channels, separated by walls, to lengthen the fluidic airpath between one inlet that is fluidically interconnected to one of the ducts 224, 226, and at the same time to increase a cooling surface that is exposed to the air channel. In another variant, chambers 266, 286 can be formed by two parallelly-arranged plates, having a plurality of parallelly-arranged baffles or walls to separate a main incoming fluid flow into a plurality of subchannels, before releasing a cooled fluid flow through outlets 267, 287. In another variant, chambers can be round or oval-shaped disk with two parallel walls, having a spirally-arranged fluidic path formed therebetween. Preferably, cooling chambers 266, 286 are made of a highly thermally conductive material, for example copper or a copper alloy, and are made to have thin walls and structures, for example a wall thickness of less than 1 mm, preferably less than 0.5 mm, more preferably less than 0.3 mm, to reduce a thermal capacity of chambers 266, 286. This allows to rapidly change a cooling temperature provided by first and second thermoelectric cooling devices 261, 281 to the air flow that will ultimately arrive at the ears of the wearer, by adjusting a cooling temperature provided by cooled surface of the respective thermoelectric cooler 264, 284, for example controlled by data processing device 248.

Outlets 267, 287 of cooling chambers 266, 286 can be fluidically connected to the earpieces 260, 280, for example by directly fluidically connecting to a respective fluid channel 268, 288 that itself can have a first and second outlet 271, 291 for delivering cooled fluid to a respective one of the ear channels of the ears of the wearer. The fluid interconnection between outlets 267, 287 can be done via a pair of ducts that lead to fluid channels 268, 288, respectively, or directly to the earpiece 260, 280 as shown, where the fluidic path from cooling chambers 266, 286 to the ear is kept short, preferably less than 5cm, to reduce an amount of reheating of the fluid on its way into a respective ear. The respective fluid channel 268, 288 can be arranged to traverse a first and second earplug or eartip 270, 290, for example a removable or fixed earplug or eartip 270, 290 that can be made of a soft, elastic, and thermally insulating material, for example silicone, that can be configured to compress and fit into a concha of each ear of a wearer, respectively. Moreover, it is possible that each earplug 270, 290 is provided with one or more air or fluid evacuation or pressure release channels 273, 293 that allows to evacuate air from the respective ear channel, to reduce a pressure that can be caused inside ear channel to the provision of a cooled air flow delivered by first and second outlet 271, 291 to the ear channel. For example, a plurality of such fluid evacuation channels 273, 293 can be arranged to be circularly around the respective fluid channel 268, 288, with an axis of extension of fluid evacuation channels 273, 293 substantially in parallel or at an oblique angle to fluid channel 268, 288.

Moreover, each one of the first and second earpieces 260, 280 can include first and second insulation elements 276, 296 for thermally insulating the first and second thermoelectric cooling devices 261, 281, from a respective one of the ears of the wearer, to increase comfort and for reducing unwanted heating or cooling of the ear or skin of wearer. For example, in the variant shown, insulation elements 276, 296 can be formed as a disk-like circular or oval structure, having a traversing bore or hole in the middle thereof, the bore or hole traversed by the respective fluid channel 268, 288 for cold air delivery, for example having a diameter of at least 5 cm or more. Ear stimulation device or system 200 for CVS can also be provided with one or more speakers or audio devices 278, 298, to combine audio, sound, or music with the CVS experience for the wearer. For example, in the variant shown, first and second speakers 278, 298 can be provided in first and second earpieces 260, 280, respectively, to deliver an audio signal to the respective fluid channel 268, 288, speakers 278, 298 shown to be embedded at least partially inside the first and second insulation elements 276, 296, respectively, for example with a speaker membrane thereof exposed to a respective fluid channel 268, 288. Fluid channel 268, 288 can thereby also operate as a sound tube for propagating sound or audio waves into the ear. Speakers 278, 298 can be powered by energy supply device 246, and controlled by data processing device 248.

In addition, device 200 can further include a cushioning element 227 arranged between the lower face of headband 220 and an area where the head of the wearer would be placed if the device 200 is worn by a wearer, the cushioning element 227 configured to isolate the head of the wearer from at least one of noise and vibration generated by the blower device 240. For example, cushioning element can be a pad, insulating sheet or layer, or a strap attached at the inner, lower face of the headband 220, for example made of a soft elastic material, for example but not limited to a neoprene sheet, rubber, polyurethane foam, memory foam.

FIG. 3 shows an overview of a system 400 for performing CVS, with a smartphone, tablet, or other computing device 300 interconnected to in-ear stimulation system or device 100, 200, configured to control system or device 100, 200, for example providing data to or receiving data from computing device 300 via a data communication link or connection, according to another aspect of the present invention. For this purpose, computing device 300 and in-ear stimulation device 200, 100 of system 400 can each be equipped with data communication interfaces for mutual data communication, and be programmed with computer code stored in data memor to execute the required data protocols for the data communication, for example but not limited to a Bluetooth communication interface, Infrared Data Association (IrDA) interface, WiFi interface, RF communication interface. Computing device 300 can be equipped with a display screen 320 with a touch sensitive interface 310 for displaying a graphical user interface (GUI) 330, graphical user interface 330 configured to display different data that have been measured and transmitted from in-ear stimulation device 100, 200 to computing device 310, for example the current temperature provided by first and second thermoelectric cooling devices 50, 60, 261, 281, data on the timely evolution of the temperature, for example during thermal charging, data of flow speeds of the fluid, data on fluid temperature, in-ear temperature, pressure value, operation status of the different elements of in-ear stimulation system or device 100, 200, for example but not limited to ventilator on/off, valves open/closed, cooling or heating on/off, audio on/off.

Computing device 300 can be configured to execute and run an application software that is designed to control the in-ear stimulation device or system 100, 200 and to provide for the graphical user interface 330, for example to provide for at least one of a temperature set value or a temperature profiles, or both for one or more of the first and second thermoelectric cooling devices 50, 60 261, 281, to provide for audio data via the data communication interface that can be played back by in-ear stimulation device or system 100, 200 with at least one of speakers first and second speakers 55, 278, 298, with the audio data being decoded by data processing device 90, 248. For example, a temperature set value, fluid flow speed value, or other value can be set within a predefined range by a graphical slider element 350, and this value can be transmitted to in-ear stimulation system 200 for execution. As another example, graphical push buttons 340 can be displayed for on/off controls, such as turning the device on and off, activating left side, right side, or both sides for providing the cooling fluid, for example by controlling valves 252, 254, activating the blower device 240. Also, GUI 330 and the application software can be configured to permit the entry and upload of a specific temperature provide to the in-ear stimulation device 200, for example a cooling profile having a changing temperature over time and having a specific duration. Also, it is possible that an alert prompt is provided to computing device 310, for example when a chosen cooling profile has ended, for example by a graphical element of GUI 330, by an auditory signal, by vibration signal, or other signal, or a combination thereof.

Also, different user profiles can be stored and managed by application software, for example different types of temperature values or profiles, different pressure values, and other data, can be associated to a specific user. It is also possible that different temperature profiles with different durations are pre-stored, or accessed from a remote server or data cloud location, for performing different types of clinic conditions. For example, it is possible that a list of several temperature profiles is displayed, associated with a name or function of the different profile, for example for treating a specific clinical condition, and the user can choose one of the different profiles from the displayed list of the GUI 330. In a variant, during or after the CVS stimulation by system or device 100, 200, it is possible that the wearer can answer questions of a feedback questionnaire, for example a health feedback questionnaire including question where the wearer can provide informative feedback on his well-being. Question of this feedback questionnaire can be displayed by GUI 330 so that the user can answer the questions on the GUI 330 by yes/no buttons 340, or comment feedback. It is also possible that in-ear stimulation system 100 of the first embodiment has a data communication interface that allows for data communication and exchange with a computing device 300 to form system 400, with the similar functionalities of the application software of computing device 300 to control the thermal cooling of the thermally conductive element 30 of earpieces 10, 20, to alert or notify the potential wearer or user via the GUI 330 of the application software, control and set desired cooling temperatures, etc.

FIG. 4 shows a side view of another embodiment of a portion of an in-ear stimulation device or system 500 in the form of a headset-type CVS device, using a central heatsink 483 that is interconnected to thermoelectric cooling element 484 via a heat transfer device 426, for example a heat pipe. In FIG. 4, only one earpiece 480 is shown for illustration purposes, but there can be another right-side earpiece (not shown). In this embodiment, a central heatsink or heat storage device 483 can be attached to the headband (not shown), at a location where the blower device 440 is attached to in-ear stimulation device or system 500. A fluidic connection from blower device 440 to earpiece 480 is provided with a duct 424 that leads to cooling chamber 486 that is formed as a porous and thermal conductive element, for example a sintered porous metal filter element, made from aluminum (Al), copper (Cu), or an alloy thereof. In turn, a tube, or duct 488 is arranged to fluidically lead away from cooling chamber 486 towards a location where the ear of the wearer would be located. At least at one side, cooling chamber 486 is integrally formed or thermally interconnected to a heating or cooling distribution plate 485, having one side thereof thermally connected or integrally formed with a heating or cooling plate of a Peltier element 484. An encapsulation 487 is formed around cooling chamber 486 to provide for fluidic or airtightness, such that air or a fluid arriving from duct 424 will pass through cooling chamber 486 and will continue to tube or duct 488. Encapsulation 487 can also be formed to cover the exposed surfaces of heating or cooling distribution plate 485 for example by using a sealing and also thermally insulating material, for example a heat-shrunk sleeve. On the other side of thermoelectric cooling element 484, a heat collector 482 can be provided that is made of a thermally conductive material, and heat collector 482 can be thermally interconnected to central heat sink 483 by a heat transfer device 426, for example a heat pipe. Blower device 440 can be configured to not only provide for a fluid flow from blower 440 to ear channel via tube, or duct 488, but can also provide for cooling air to central heat sink 483. Heat collector 482, thermoelectric cooling element 484 and heat transfer device 426 can be at least partially covered or encapsulated into a casing or other thermally-insulating material. For example, it is possible that heat transfer device and heat collector 482 are arranged inside the headband (not shown).

With the herein described devices and systems 100, 200, 300, 400, 500 it is possible to perform CVS for different types of therapeutic purposes, having a compact and easy-to-use portable design. For example, it is possible effectively and efficiently to treat various clinical conditions including sleep difficulties, mood disorders, chronic pain, and issues with memory loss, and memory performance. See Grabherr et al., "The Moving History of Vestibular Stimulation as a Therapeutic Intervention," Multisensory Research, Vol. 28, No. 5-6, year 2015, pp. 653-687.

According to another aspect of the present invention, the herein presented devices and systems 100, 200, 300, 400, 500, in combination with one or more speaker devices 55, or first and second speakers 278, 298 can be used as an audio or sound portable earphone or headphone with a primary function to provide for audio or sound, and with a secondary function to cool the ear channel of wearer for a more pleasant music or audio experience, for example for audio or sound sessions of long duration where the heating of the ear of the wearer needs to be avoided such a gaming, music studio applications, call center operators. In this respect, the CVS function may or may not be present, or can only presented as an option, for example controllable by application software of computing device 300.

While the invention has been disclosed with reference to certain preferred embodiments, numerous modifications, alterations, and changes to the described embodiments, and equivalents thereof, are possible without departing from the sphere and scope of the invention. Accordingly, it is intended that the invention not be limited to the described embodiments, and be given the broadest reasonable interpretation in accordance with the language of the appended claims.

## Claims

1. An in-ear stimulation system (100) for caloric vestibular stimulation (CVS) to an ear of a wearer, the system comprising at least one earpiece (10, 20) and a device (80) for holding the at least one earpiece (10, 20),
wherein the at least one earpiece (10, 20) includes,
a thermally conductive element (30) having a contact surface (32) and a protrusion (36) configured to fit at least partially into an ear channel of the ear of the wearer, and
wherein the device (80) for holding the at least one earpiece (10, 20) includes,
a thermoelectric cooler or heater (50, 60) having a cooling or a heating surface (52, 62), and
an energy supply device (70) for providing electric energy to the thermoelectric cooler or heater (50, 60).

2. The in-ear stimulation system (100) according to claim 1, further comprising:
a securing element (92, 94) for securing the at least one earpiece (10, 20) to the device (80) such that the contact surface (32) of the thermally conductive element (30) of the at least on earpiece (10, 20) is in contact with the cooling surface (52, 62) of the thermoelectric cooler or heater (50, 60), when the at least one earpiece (10, 20) is placed to the device (80).

3. The in-ear stimulation system (100) according to claim 1, further comprising:
a speaker device (55) for providing an audio signal to the ear of the wearer.

4. The in-ear stimulation system (100) according to claim 1, wherein the thermally conductive element (30) further includes a thermal energy storage volume (34) that arranged in thermal communication between the contact surface (32) and the protrusion (36).

5. The in-ear stimulation system (100) according to claim 1, wherein the at least one earpiece (10, 20) includes an outer shell (12) and a thermal insulation layer (40), the thermal insulation layer (40) arranged between the thermally conductive element (30) and the outer shell (12).

6. The in-ear stimulation system (100) according to claim 1, wherein the protrusion (36) of the thermally conductive element (30) includes a bore (38) at least partially traversing the protrusion (36) or includes a bore (138) that is fully traversing the protrusion (36) and a head part of the at least one earpiece (10, 20), thereby forming an air inlet (133) and an air outlet (135).

7. The in-ear stimulation system (100) according to claim 6, further comprising:
a fan device (155) and an energy supply device (157) for powering the fan device (155), the fan device (155) configured to provide for an airflow from the air inlet (133) to the air outlet (135).

8. The in-ear stimulation system (100) according to claim 1, wherein the holder (80) includes a temperature sensor (96, 98) for measuring a temperature of at least one of the thermoelectric cooler or heater (50, 60) or the thermally conductive element (30).

9. An ear stimulation device (200) for caloric vestibular stimulation (CVS) to an ear of a wearer, comprising:
a headband (220);
first and second earpieces (260, 280) held by the headband (220);
a blower device (240) arranged at the headband (220);
first and second fluidic ducts (224, 226) providing for a fluidic connection between the blower device (240) and the two earpieces (260, 280);
first and second thermoelectric cooling or heating devices (261, 281) fluidically arranged between the blower device (240) and the two earpieces (260, 280) configured to cool a fluid arriving from the blower device (240).

10. The ear stimulation device (200) of claim 9, wherein each one of the first and second thermoelectric cooling devices (261, 281) includes:
a thermoelectric cooler (264, 284);
a heat sink (262, 282) having a first surface in contact with a heated side of the thermoelectric cooling or heating devices (261, 281), and a second surface exposed to an external environment; and
a cooling chamber (266, 286) having a fluid inlet for receiving an incoming fluid from the first and second fluidic ducts (224, 226), and a fluid outlet for releasing a cooled outgoing fluid towards the earpiece (260, 280).

11. The ear stimulation device (200) of claim 9, wherein the blower device (240) is arranged at the headband (220) to be substantially halfway between the first and second earpieces (260, 280), configured to draw in the fluid from an external environment.

12. The ear stimulation device (200) of claim 9, wherein first and second earpieces (260, 280) each include:
a fluid channel (268, 288) in fluidic communication with an outlet of a respective one of the first and second thermoelectric cooling or heating devices (261, 281) and having an outlet (271, 291) for delivering cooled fluid to a respective one of the ears of the wearer.

13. The ear stimulation device (200) of claim 9, wherein first and second earpieces (260, 280) each include:
an insulation element (276, 296) for thermally insulating the first and second thermoelectric cooling or heating devices (261, 281) from a respective one of the ears of the wearer.

14. The ear stimulation device (200) of claim 13, wherein the first and second earpieces (260, 280) each include:
first and second speakers (278, 298) for providing audio signals to a respective one of the fluid channels (268, 288).

15. The ear stimulation system (400) comprising:
a data processing device (300) having a first data communication interface and a display screen (320), and
an ear stimulation device (100, 200) for caloric vestibular stimulation (CVS) to an ear of a wearer, the ear stimulation device (100, 200) including,
at least one earpiece (10, 20, 260, 280, 480),
means for cooling or heating an ear channel of a wearer (30, 261, 281, 484), and
a second data communication interface for communicating with the first data communication interface of the data processing device (300),
wherein the data processing device (300) is configured provide for at least one of a temperature set value or a temperature profile to the ear stimulation device (100, 200) for cooling or heating an ear channel of a wearer.
